# FASCICULE DE BREVET EUROPEEN

(11) **EP 1 353 630 B1**
(45) Date de publication et mention de la délivrance du brevet: **11.11.2009**
(21) Numéro de dépôt: 02711953.6
(22) Date de dépôt: 14.01.2002
(51) Int. Cl.: A61K 8/87, A61K 8/81, A61K 8/73, A61Q 1/00, A61Q 1/10

(54) **COMPOSITION COSMETIQUE COMPRENANT UN MELANGE DE POLYMERES**
KOSMETISCHE ZUSAMMENSETZUNG ENTHALTEND EIN POLYMERGEMISCH
COSMETIC COMPOSITION COMPRISING A MIXTURE OF POLYMERS

(30) Priorité: 15.01.2001 FR 0100479
(43) Date de publication de la demande: 22.10.2003
(73) Titulaire: L'ORÉAL, 75008 Paris (FR)
(72) Inventeur: COLLIN, Nathalie, F-92330 Sceaux (FR)
(74) Mandataire: Boulard, Denis
(86) Numéro de dépôt international: PCT/FR2002/000129
(87) Numéro de publication internationale: WO 2002/055031

(56) Documents cités:
- EP-A- 1 064 919
- EP-A- 1 064 920
- US-A- 4 871 536

## Description

La présente invention a pour objet une composition de maquillage comprenant un mélanges de polymères particuliers pour obtenir un maquillage rapide des matières kératiniques. L'utilisation selon l'invention est plus particulièrement destinée aux fibres kératiniques, notamment sensiblement longitudinales, d'êtres humains tels que les cils, les sourcils et les cheveux ou bien encore les faux-cils ou les postiches. Plus spécialement, la composition est un mascara. Par mascara, on entend une composition de maquillage des cils, une base de maquillage, un produit à appliquer sur un maquillage, dit encore top-coat, ou bien encore un produit de traitement cosmétique des cils.

Les compositions de revêtement des cils, appelées mascara, comprennent généralement, de façon connue, au moins une cire et au moins un polymère filmogène pour déposer un film de maquillage sur les cils et gainer ces derniers, comme le décrit par exemple les documents WO-A-91/12793 et WO-A-95/15741. Les utilisatrices attendent pour ces produits de bonnes propriétés cosmétiques telles que l'adhérence sur les cils, un allongement ou un recourbement des cils, ou bien encore une bonne tenue du mascara dans le temps, notamment une bonne résistance aux frottements par exemple des doigts ou des tissus (mouchoirs, serviettes). Il est notamment connu du document FR-A-2528699 un mascara contenant des cires et une association de polymère anionique et de polymère cationique.

Toutefois, avec ces compositions, les propriétés de maquillage comme le gainage, l'allongement ou le recourbement des cils sont obtenus lorsqu'une quantité importante de produit est déposée sur les cils à l'aide d'un applicateur, telle qu'une brosse à mascara. L'utilisatrice doit donc appliquer plusieurs fois la brosse imprégnée de produit sur les cils, ce qui demande de consacrer un certain temps pour se maquiller et obtenir les résultats de maquillage souhaités. Or ce temps peut être perçu comme beaucoup trop long par les utilisatrices pressées. Un besoin existe donc de disposer de mascaras permettant d'obtenir rapidement et facilement le maquillage attendu.

Le but de la présente invention est de disposer d'une composition de maquillage des matières kératiniques, notamment des fibres kératiniques tels que les cils, s'appliquant facilement sur les matières kératiniques et conduisant rapidement à un maquillage présentant de bonnes propriétés cosmétiques.

Les inventeurs ont constaté de façon surprenante que l'utilisation d'un polymère non ionique à hétéroatome particulier dans une composition cosmétique contenant un polymère anionique et un polymère cationique permet d'améliorer les propriétés d'adhérence de la composition sur les matières kératiniques, notamment sur les fibres kératiniques comme les cils. La composition s'applique facilement sur les matières kératiniques et permet de déposer rapidement la composition en quantité suffisante pour obtenir un maquillage présentant les propriétés cosmétiques attendues. En particulier, on obtient rapidement un dépôt épais du maquillage sur les matières kératiniques ce qui évite aux utilisatrices d'appliquer trop longtemps la composition sur les matières kératiniques. La composition permet donc un maquillage rapide (ou maquillage "express") des matières kératiniques. Ainsi, pour un mascara, on obtient un maquillage qui épaissit rapidement les fibres kératiniques, notamment les cils ; on constate ainsi une charge instantanée des cils.

De façon plus précise, l'invention a pour objet une composition, notamment de maquillage ou de soin des matières kératiniques, comprenant, dans un milieu physiologiquement acceptable contenant une phase grasse, :
- (i) un premier polymère de masse moléculaire moyenne en poids inférieure à 100 000, choisi parmi les polymères de formule (I') de la revendication 1 et leurs mélanges,
- (ii) un polymère filmogène anionique,
- (iii) un polymère filmogène cationique,
lesdits polymères filmogène anionique et cationique étant différents dudit premier polymère.

L'invention a également pour objet un procédé cosmétique de maquillage ou de soin non thérapeutique des matières kératiniques, notamment des fibres kératiniques telles que les cils, comprenant l'application sur lesdites matières kératiniques d'une composition telle que définie précédemment.

L'invention a aussi pour objet l'utilisation, d'une composition telle que définie précédemment, pour obtenir un maquillage rapide des matières kératiniques.

Par milieu physiologiquement acceptable, on entend un milieu non toxique et susceptible d'être appliquée sur la peau, les phanères ou les lèvres d'êtres humains, comme un milieu cosmétique.

Par "polymère", on entend au sens de l'invention un composé ayant au moins 2 motifs de répétition, et de préférence au moins 3 motifs de répétition, qui sont identiques.

Le premier polymère de la composition selon l'invention présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000 ,et mieux de 2000 à 10 000.

Ces premiers polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone. Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I') suivante : 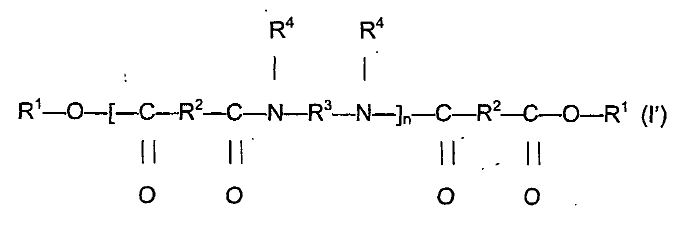 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

Dans le cas particulier de la formule (I'), les chaînes grasses terminales éventuellement fonctionnalisées au sens de l'invention sont des chaînes terminales liées au dernier hétéroatome, ici l'azote, du squelette polyamide.

En particulier, les groupes ester de la formule (I'), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux au moins 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (I') se présentent sous forme de mélanges de polymères, ces mélanges pouvant en outre contenir un produit de synthèse correspondant à un composé de formule (I') où n vaut 0, c'est-à-dire un diester.

A titre d'exemple de premiers polymères selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C: Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme premier polymère utilisable dans l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Le premier polymère présent dans la composition selon l'invention a avantageusement une température de ramollissement supérieure à 65°C et pouvant aller jusqu'à 190°C. De préférence, il présente une température de ramollissement allant de 70 à 130°C et mieux de 80 à 105°C. Le premier polymère est en particulier un polymère non cireux.

Ce premier polymère présentent du fait de leur(s) chaîne(s) grasse(s), une bonne solubilité dans les huiles et donc conduisent à des compositions macroscopiquement homogènes même avec un taux élevé (au moins 25%) de polymère, contrairement à des polymères exempts de chaîne grasse.

Le premier polymère peut être présent dans la composition selon l'invention en une teneur allant de 0,01 % à 10 % en poids, par rapport au poids total de la composition, de préférence allant de 0,05 % à 5 % en poids, et mieux allant de 0,1 % à 3 % en poids.

Selon l'invention, la composition selon l'invention peut contenir tout polymère filmogène anionique, de préférence non réticulé, ou cationique connu en soi. Ces polymères peuvent être utilisés sous forme solubilisée ou sous forme de dispersions aqueuses de particules solides de polymère. Le polymère cationique et le polymère anioonique sont différents du premier polymère décrit précédemment. Dans la présente demande, on entend par "polymère filmogène", un polymère apte à former à lui seul ou en présence d'un agent auxiliaire de filmification, un film continu et adhérent sur un support, notamment sur les matières kératiniques.

Les polymères anioniques généralement utilisés peuvent être des polymères comportant des groupements dérivés d'acide carboxylique, sulfonique ou phosphorique et peuvent avoir un poids moléculaire moyen en poids compris entre environ 500 et 5.000.000, et de préférence supérieur à 100000 et inférieur ou égal à 5.000.000.
1) Les groupements carboxyliques peuvent être apportés par des monomères mono ou diacides carboxyliques insaturés tels que ceux répondant à la formule (I) suivante : 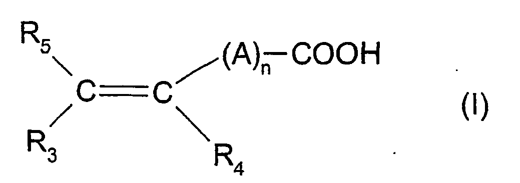 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle ;
   Dans la formule précitée un radical alkyle inférieur désigne de préférence un groupement ayant 1 à 4 atomes de carbone et en particulier, méthyle et éthyle.
   Les polymères anioniques à groupements carboxyliques préférés selon l'invention sont :
   A) les homo- ou copolymères d'acide acrylique ou méthacrylique ou leurs sels (notamment sels de métaux alcalins ou alcalino-terreux, d'ammoniums) et en particulier les produits commercialisés sous les dénominations VERSICOL E ou K par la société ALLIED COLLOID, ULTRAHOLD par la société BASF, DARVAN 7 par la société VANDERBILT.
   B) les copolymères des acides acrylique ou méthacrylique avec un monomère monoéthylénique tel que l'éthylène, le styrène, les esters vinyliques, les esters d'acide acrylique ou méthacrylique. Ces copolymères peuvent être greffés sur un polyalkylène glycol tel que le polyéthylène glycol. De tels polymères sont décrits en particulier dans le brevet français 1.222.944 et la demande allemande 2.330.956. On peut notamment citer les copolymères comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut citer aussi les copolymères d'acide acrylique et d'acrylamide commercialisés sous la forme de leur sel de sodium sous les dénominations RETEN 421, 423 ou 425 par la Société HERCULES. On peut également citer les copolymères d'acide acrylique et de méthacrylate d'alkyle en C₁-C₄ et les terpolymères de vinylpyrrolidone, d'acide (méth)acrylique et de (méth)acrylate d'alkyle en C₁-C₂₀ par exemple de lauryle (tel que celui commercialisé par la société ISP sous la dénomination ACRYLIDONE LM), de tertiobutyle (LUVIFLEX VBM 70 commercialisé par BASF) ou de méthyle (STEPANHOLD EXTRA commercialisé par STEPAN) et les terpolymères acide méthacrylique/ acrylate d'éthyle/ acrylate de tertiobutyle tel que le produit commercialisé sous la dénomination LUVIMER 100 P par la société BASF.
   C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 commercialisées par la société NATIONAL STARCH.
   D) les copolymères dérivés d'acides ou d'anhydrides carboxyliques monoinsaturés en C₄-C₈ choisis parmi :
      - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux commercialisés sous les dénominations GANTREZ AN ou ES, AVANTAGE CP par la société ISP.
      - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
         Ces polymères sont par exemple décrits dans les brevets français 2.350.384 et 2.357.241 de la demanderesse.
   E) les polyacrylamides comportant des groupements carboxylates,
   F) les sels de sodium des acides polyhydroxycarboxyliques.
      - et leurs mélanges.
2) Les polymères comprenant les groupements sulfoniques peuvent être des polymères comportant des motifs vinylsulfonique, styrène sulfonique, naphtalène sulfonique, acrylamido alkylsulfonique, ou bien encore des polyesters sulfoniques.
   Ces polymères peuvent être notamment choisis parmi :
   - les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone ;
   - les sels de l'acide polystyrène sulfonique les sels de sodium ayant un poids moléculaire moyen en poids d'environ 500.000 et d'environ 100.000 commercialisés respectivement sous les dénominations Flexan 500 et Flexan 130 par National Starch. Ces composés sont décrits dans le brevet FR 2.198.719 ;
   - les sels d'acides polyacrylamide sulfoniques ceux mentionnés dans le brevet US 4.128.631 et plus particulièrement l'acide polyacrylamidoéthylpropane sulfonique commercialisé sous la dénomination COSMEDIA POLYMER HSP 1180 par Henkel ;
   - les polyesters sulfoniques portant au moins un groupement -SO₃M avec M représentant un atome d'hydrogène, un ion ammonium NH₄⁺ ou un ion métallique. Le copolyester peut être par exempte un copolymère d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique.

   L'acide dicarboxylique peut être choisi parmi l'acide phtalique, l'acide isophtalique, l'acide téréphtalique. Le diol peut être choisi parmi l'éthylène glycol, le diéthylène glycol, le triéthylène glycol, le 1,3-propanediol, le 1,4-cyclohexane diméthanol, le 1,4-butanédiol. Le monomère aromatique bifonctionnel portant le groupement - SO₃M peut être choisi parmi l'acide sulfoisophtalique, notamment le sel de sodium de l'acide 5-sulfo-isophtalique, l'acide sulfotéréphtalique, l'acide sulfophtalique, l'acide 4-sulfonaphtalène-2,7-dicarboxylique.
   Comme polyester préféré, on peut utiliser un polyester consistant essentiellement en des unités répétées d'acide isophtalique, de diol et d'acide sulfo-isophtalique, et notament les sulfopolyesters obtenus par condensation de di-éthylèneglycol, de cyclohexane di-méthanol, d'acide isophtalique, d'acide sulfoisophtalique. Comme polyester sulfonique, on peut utiliser ceux commercialisés sous les dénominations AQ55S, AQ 38S, AQ 29S par la société EASTMAN.
   On peut également employer comme polymère anionique l'acide (désoxy)ribonucléique.
   Selon l'invention, les polymères anioniques sont de préférence choisis parmi les copolymères d'acide acrylique tels que les terpolymères acide acrylique / acrylate d'éthyle / N-tertiobutylacrylamide commercialisés notamment sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/ néododécanoate de vinyle commercialisés notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés commercialisés par exemple sous la dénomination GANTREZ par la société ISP, les copolymères d'acide méthacrylique et de méthacrylate de méthyle commercialisés sous la dénomination EUDRAGIT L par la société ROHM PHARMA, les copolymères d'acide méthacrylique/ méthacrylate de méthyle / acrylate d'alkyle en C1-C4 / acide acrylique ou méthacrylate d'hydroxyalkyle en C1-C4 commercialisés sous forme de dispersions sous la dénomination AMERHOLD DR 25 par la société AMERCHOL ou sous la dénomination ACUDYNE 255 par la société ROHM & HAAS, les copolymères d'acide méthacrylique et d'acrylate d'éthyle commercialisés sous la dénomination LUVIMER MAEX ou MAE par la société BASF et les copolymères acétate de vinyle/acide crotonique, les copolymères acétate de vinyle/acide crotonique greffés par du polyéthylèneglycol sous la dénomination ARISTOFLEX A par la société BASF, les homopolymères d'acide acrylique ou méthacrylique commercialisés par exemple sous la dénomination VERSICOL E 5 ou lé polyméthacrylate de sodium vendu sous la dénomination DARVAN 7 par la société VANDERBILT, et leurs mélanges.
   Les polymères anioniques les plus particulièrement préférés sont choisis parmi les polymères anioniques non réticulés comme les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5 OU le polyméthacrylate de sodium vendu sous la dénomination DARVAN 7 par la société VANDERBILT, et leurs mélanges.
   Selon l'invention, on peut également utiliser des polymères anioniques sous forme de latex ou de pseudolatex, c'est à dire sous forme d'une dispersion de particules de polymères insolubles.
3) Selon l'invention, on peut également utiliser les polymères anioniques de type siliconés greffés comprenant une portion polysiloxane et une portion constituée d'une chaîne organique non-siliconée, l'une des deux portions constituant la chaîné principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 1 à 40% en poids d'acide acrylique ;
c) 5 à 40% en poids de macromère siliconé de formule (II): 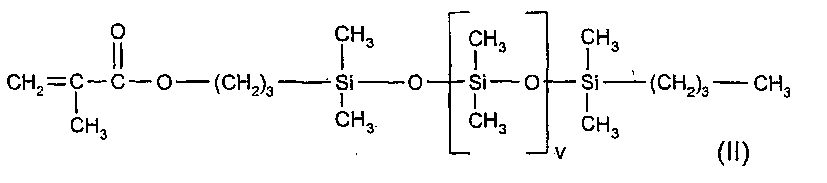 avec v étant un nombre allant de 5 à 700 ; les pourcentages en poids étant calculés par rapport au poids total des monomères.

Une famille de polymères siliconés à squelette polysiloxanique greffé par des monomères organiques non siliconés convenant particulièrement bien à la mise en oeuvre de la présente invention est constituée par les polymères siliconés comportant dans leur structure le motif de formule (III) suivant : 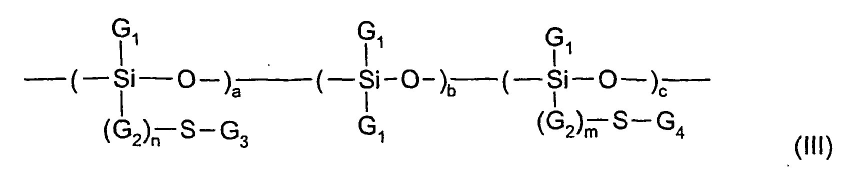 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (III) ci-dessus présente au moins l'une, et encore plus préférentiellement l'ensemble, des caractéristiques suivantes :
- les radicaux G₁ désignent un radical alkyle en C₁-C₁₀ de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle en C₁-C₁₀ , de préférence le (méth)acrylate d'isobutyle ou de méthyle.

De préférence, le motif de formule (III) ci-dessus peut également présenter l'ensemble des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- c est égal zéro.

Des exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle tel que le poly-(méth)acrylate d'isobutyle.
On utilise particulièrement les polymères siliconés greffés de formule (III) de structure polyméthyl/méthylsiloxane à groupements propyl thio-3 acide polyméthacrylique et groupements propyl thio-3 polyméthacrylate de méthyle et les polymères siliconés greffés de formule (III) de structure polyméthyl/méthylsiloxane à groupements propyl thio-3 acide polyacrylique.

Selon l'invention, le ou les polymères anioniques peuvent être présents en une teneur allant de 0,01 % à 20 % en poids, de préférence de 0,05 % à 15 % en poids, et encore plus préférentiellement de 0,1 % à 7 % en poids, du poids total de la composition.

Les polymères cationiques utilisables conformément à la présente invention peuvent être choisis parmi tous ceux déjà connus en soi notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ, et mieux supérieure à 100000 et inférieure ou égale à 3.10⁶.

Parmi les polymères cationiques, on peut citer plus particulièrement les polymères du type polyamine, polyaminoamide et polyammonium quaternaire. Ce sont des produits connus.

Une famille de polymères cationiques est celle des polymères cationiques siliconés. Parmi ces polymères, on peut citer :
(a) les polymères siliconés répondant à la formule (IV) suivante :

   R⁶ₐG⁵₃₋ₐ-Si(OSiG⁶₂)ₙ-(OSiG⁷_{b}R⁷_{2-b})ₘ-O-SiG⁸₃₋ₐ-R⁸_{a'} (IV)

   dans laquelle :
   G⁵, G⁶, G⁷ et G⁸, identiques ou différents, désignent un atome d'hydrogène, un groupement phényle, OH, alkyle en C₁-C₁₈, par exemple méthyle, alcényle en C₂-C₁₈, ou alcoxy en C₁-C₁₈
   a, a', identiques ou différents, désignent le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R⁶, R⁷, R⁸, identiques ou différents, désignent un radical monovalent de formule - C_{q}H_{2q}Oₛ R⁹ₜL dans laquelle q est un nombre de 1 à 8, s et t, identiques ou différents, sont égaux à 0 ou à 1, R⁹ désigne un groupement alkylène éventuellement hydroxylé et L est un groupement aminé éventuellement quaternisé choisi parmi les groupements :
      -NR"-CH₂-CH₂-N'(R")₂
      -N(R")₂
      -N^{⊕}(R")₃ A⁻
      -N^{⊕}H(R")₂ A⁻
      -N^{⊕}H₂(R") A⁻
      -N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A- représente un ion halogénure tel que par exemple fluorure, chlorure, bromure ou iodure.
   Des produits correspondant à cette définition sont par exemple les polysiloxanes dénommés dans le dictionnaire CTFA "amodiméthicone" et répondant à la formule (V) suivante : 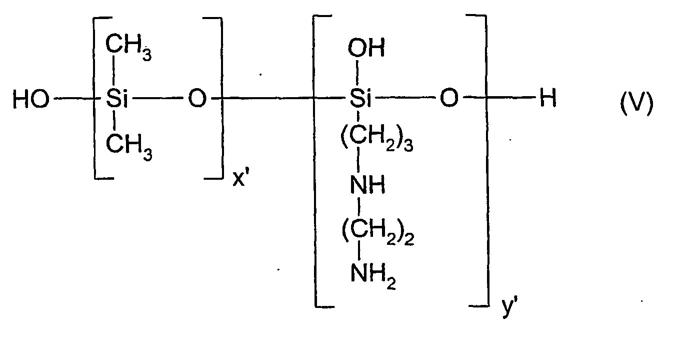 dans laquelle x' et y' sont des nombres entiers dépendant du poids moléculaire, généralement tels que ledit poids moléculaire est compris entre 5 000 et 20 000 environ.
   Un produit correspondant à la formule (IV) est le polymère dénommé dans le dictionnaire CTFA "triméthylsilylamodiméthicone", répondant à la formule (VI) : 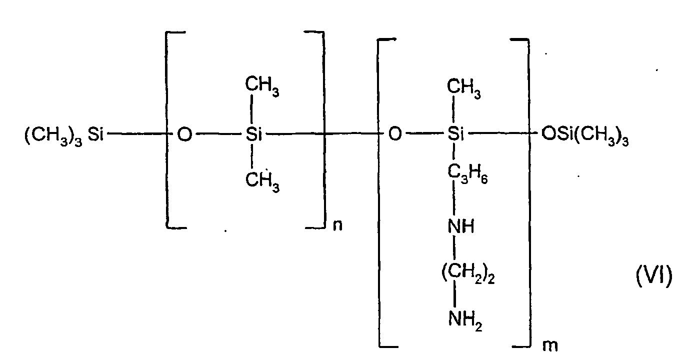 dans laquelle n et m ont les significations données ci-dessus pour la formule (IV).
   Un produit commercial répondant à cette définition est un mélange (90/10 en poids) d'un polydiméthylsiloxane à groupements aminoéthyl aminoisobutyle et d'un polydiméthylsiloxane commercialisé sous la dénomination Q2-8220 par la société DOW CORNING.
   De tels polymères sont décrits par exemple dans la demande de brevet EP-A-95238.
   D'autres polymères répondant à la formule (IV) sont les polymères siliconés répondant à la formule suivante (VII) : 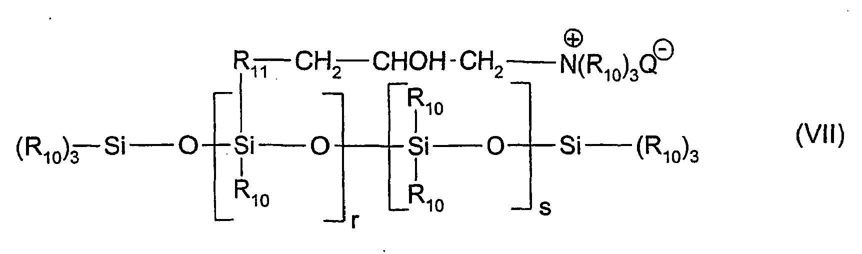 dans laquelle :
   R₁₀ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₁₁ représente un radical hydrocarboné divalent, notamment un radical alkylène en C₁-C₁₈ ou un radical alkylèneoxy divalent en C₁-C₁₈, par exemple en C₁-C₈ ;
   Q- est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

   De tels polymères sont décrits plus particulièrement dans le brevet US 4 185 087.
(b) les composés de formule : NH-[(CH₂)₃-Si[OSi(CH₃)₃]]₃ correspondant à la dénomination CTFA "aminobispropyldiméthicone".

Un polymère entrant dans cette classe est le polymère commercialisé par la Société Union Carbide sous la dénomination "Ucar Silicone ALE 56.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit commercialisé sous la dénomination "mulsion Cationique DC 929" par la Société DOW CORNING qui comprend, outre l'amodiméthicone, un agent de surface cationique comprenant un mélange de produits répondant à la formule (VIII) : 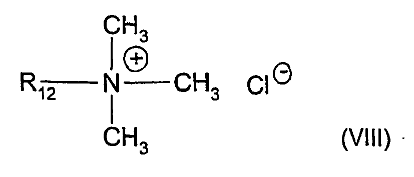 dans lequel R₁₂ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40, un autre agent de surface non ionique de formule : C₁₂H₂₅-(OCH₂-CH₂)ₙ-OH où n = 6 encore dénommé isolaureth-6, et du glycol.

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits commercialisés sous la dénomination "Gafquat^{®}" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose, notamment des hydroxyalkyl(C1-C4) cellulose, comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde (notamment épichlorhydrine) substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylmidopropyl triméthyl ammonium ou de diméthyldiallylammonium.
   Les produits commercialisés répondant à cette définition sont plus particulièrement les produits commercialisés sous la dénomination "Celquat L 200" et "Celquat H 100" par la Société National Starch.
(4) Les polysaccharides cationiques décrits plus particulièrement dans les brevets US 3 589 578 et 4 031 307 et plus particulièrement le produit commercialisé sous la dénomination "Jaguar C.13 S" commercialisé par la Société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques, suivie d'une alcoylation par des agents bifonctionnels. On peut citer par exemple les polymères acide adipique-diacoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine commercialisés sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques éliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères de méthyl diallyl amine ou de diallyl diméthyl ammonium tels que les homopolymères ou les copolymères comportant comme constituant principal de la chaîne des motifs répondant aux formules (IX) ou (IX') : 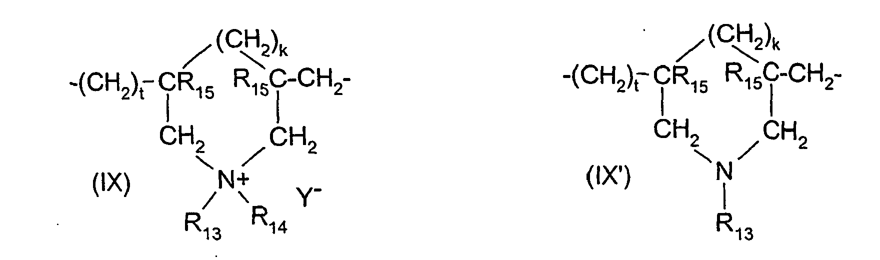 formules dans lesquelles k et t sont égaux à 0 ou 1, la somme k + t étant égale à 1 ; R₁₅ désigne un atome d'hydrogène ou un radical méthyle ; R₁₃ et R₁₄, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₃ et R₁₄ peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   On peut citer par exemple l'homopolymère de chlorure de diallyldiméthylammonium commercialisé sous la dénomination "MERQUAT 100" par la société MERCK et les copolymères de chlorure de diallyldiméthylammonium et d'acrylamide commercialisés sous la dénomination "MERQUAT 550".
(10) le polymère de diammonium quaternaire contenant des motifs récurants répondant à la formule (X) 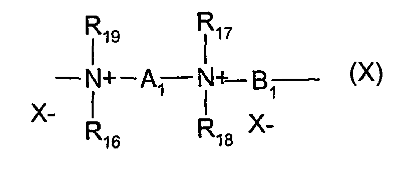 formule (X) dans laquelle
   R₁₆, R₁₇, R₁₈ et R₁₉, identiques ou différents, représentent des radicaux aliphatiques, alicycliques, ou arylaliphatiques contenant de 1 à 20 atomes de carbone ou des radicaux hydroxyalkylaliphatiques inférieurs, ou R₁₆, R₁₇, R₁₈ et R₁₉, ensemble ou séparément, constituent avec les atomes d'azote auxquels ils sont rattachés des hétérocycles contenant éventuellement un second hétéroatome autre que l'azote ou bien R₁₆, R₁₇, R₁₈ et R₁₉, représentent un radical alkyle en C₁-C₆ linéaire ou ramifié substitué par un groupement nitrile, ester, acyle, amide ou -CO-O-R₂₀-D ou -CO-NH-R₂₀-D où R₂₀ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique ;
   A1, R₁₆ et R₁₈ peuvent former avec les deux atomes d'azote auxquels ils sont rattachés un cycle pipérazinique ; en outre si A₁ désigne un radical alkylène
      ou hydroxyalkylène, linéaire ou ramifié, saturé ou insaturé, B₁ peut également désigner un groupement (CH2)ₙ-CO-D-OC-(CH2)ₙ-
      dans lequel n désigne un entier allant de 1 à 6, D désigne :
      a) un reste de glycol de formule : -O-Z-O-, où Z désigne un radical hydrocarboné linéaire ou ramifié ou un groupement répondant à l'une des formules suivantes :
         -(CH₂-CH₂-O)x-CH₂-CH₂-

         -[CH₂-CH(CH₃)-O]_{y}-CH₂-CH(CH₃)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

      De préférence, X- est un anion tel que le chlorure ou le bromure.
      Ces polymères ont une masse moléculaire moyenne en nombre généralement comprise entre 1000 et 100000. Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (XI) : 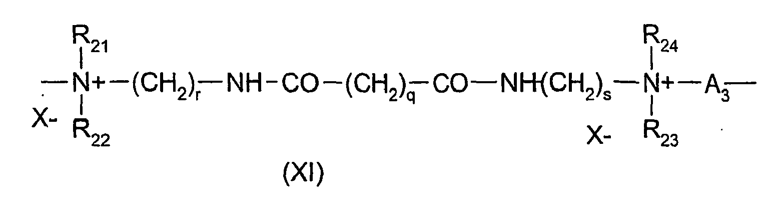 formule dans laquelle :
   R₂₁, R₂₂, R₂₃ et R₂₄, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
      où p est égal à 0 ou à un nombre entier compris entre 1 et 6, sous réserve que R₂₁, R₂₂, R₂₃ et R₂₄ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A₃ désigne un radical d'un dihalogénure ou représente de préférence -CH₂-CH₂-O-CH₂-CH₂-.
      De tels composés sont notamment décrits dans la demande de brevet EP-A-122 324.
      On peut par exemple citer parmi ceux-ci, les produits "Mirapol^{®} A 15", "Mirapol^{®} AD1", "Mirapol^{®} AZ1" et "Mirapol^{®} 175" commercialisés par la société Miranol.
(12) les homopolymères ou copolymères dérivés des acides acrylique ou méthacrylique et comportant des motifs de formules (XII), (XIII), (XIV) suivants : 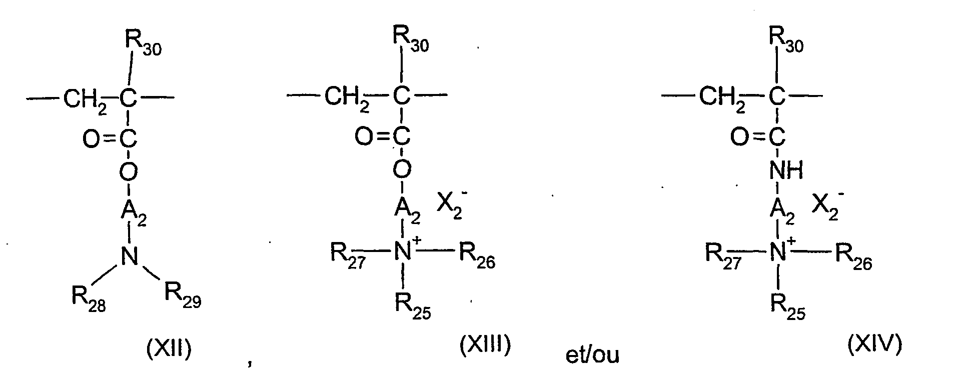 dans lesquels les groupements R₃₀ désignent indépendamment H ou CH₃,
   les groupements A₂ désignent indépendamment un groupe alcoyle linéaire
   ou ramifié de 1 à 6 atomes de carbone ou un groupe hydroxyalcoyle de 1 à 4 atomes de carbone,
   les groupements R₂₅, R₂₆, R₂₇, identiques ou différents, désignant indépendamment un groupe alcoyle de 1 à 18 atomes de carbone ou un radical benzyle,
   les groupements R₂₈ et R₂₉ représentent un atome d'hydrogène ou un groupement alcoyle de 1 à 6 atomes de carbone,
   X₂⁻ désigne un anion, par exemple méthosulfate ou halogénures, tel que chlorure ou bromure.
   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations LUVIQUAT^{®} FC 905, FC 550 et FC 370 par la société BASF.
(14) Les polyamines comme le Polyquart H commercialisé par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyl triméthyl ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contentant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE^{®} SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les copolymères du chlorure de diméthyldiallylammonium et d'acrylamide ayant un poids moléculaire supérieur à 500 000, commercialisés sous les dénominations « MERQUAT^{®} 550 » et « MERQUAT^{®} S » par la Société MERCK, les polysaccharides cationiques et plus particulièrement le polymère commercialisé sous la dénomination « JAGUAR^{®} C13S » par la Société MEYHALL, et les polyaminoamides de la famille (6) décrits ci-dessus.

Selon l'invention, on peut également utiliser des polymères cationiques sous forme de latex ou de pseudolatex, c'est à dire sous forme d'une dispersion de particules de polymères insolubles.

Selon l'invention, le ou les polymères cationiques peuvent être présents en une teneur allant de 0,01 % à 20 % en poids, de préférence de 0,01 % à 15 % en poids, et encore plus préférentiellement de 0,05 % à 5 % en poids, du poids total de la composition.

Le rapport charge cationique du(es) polymère(s) cationique(s) / charge anionique du(es) polymère(s) anionique(s) exprimée en meq./g est généralement compris entre 0,25 et 5, de préférence entre 0,5 et 2 et encore plus préférentiellement entre 0,75 et 1,25.

La charge cationique est le nombre d'atome d'amine quaternaire, tertiaire, secondaire ou primaire par gramme de polymère.

Avantageusement, le polymère cationique peut être une hydroxyalkyl(C1-C4)cellulose comportant des groupements ammonium quaternaires, notamment une hydroxyéthylcellulose réticulée à l'épichlorhydrine quaternisée par la triméthylamine ; le polymère anionique peut être un polyméthacrylate de sodium.

La phase grasse de la composition peut comprendre des corps gras choisis parmi les huiles, les solvants organiques, les cires, les corps gras pâteux, et leurs mélanges. La phase grasse peut former une phase continue de la composition.

La phase grasse peut notamment être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange, dans la mesure où elles forment un mélange homogène et stable et où elles sont compatibles avec l'utilisation envisagée.

La phase grasse totale de la composition peut représenter de 1 % à 99 % en poids, par rapport au poids total de la composition, et de préférence de 5 à 85 % en poids.

Avantageusement, la phase grasse de la composition peut comprendre au moins une huile ou solvant organique volatile et/ou au moins une huile non volatile.

Par " huile ou solvant organique volatile", on entend au sens de l'invention tout milieu non aqueux susceptible de s'évaporer au contact de la peau en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 10⁻² à 300 mm de Hg (0,13 Pa à 40.000 Pa) et de préférence supérieure à 0,3 mm de Hg (30 Pa). Par "huile non volatile", on entend une huile restant sur la peau à température ambiante et pression atmosphérique au moins plusieurs heures et ayant notamment une pression de vapeur inférieure à 10⁻² mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0 % à 98 % en poids (notamment de 0,1 % à 98 %), par rapport au poids total de la composition, de préférence de 0 % à 65 % en poids (notamment de 1 % à 65%).

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ; et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0 à 80 % (notamment de 0,1 à 80 %) en poids, de préférence de 0 % à 50 % en poids (notamment 0,1 à 50 %), par rapport au poids total de la composition, et mieux de 0 % à 20 % en poids (notamment 0,1 % à 20 %).

La phase grasse de la composition selon l'invention peut comprendre une cire. Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200° C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique. On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone ou les cires fluorées.

Les cires présentes dans la composition peuvent être dispersées sous forme de particules dans un milieu aqueux. Ces particules peuvent avoir une taille moyenne allant de 50 nm à 10 µm, et de préférence de 50 nm à 3,5 µm.
En particulier, la cire peut être présente sous forme d'émulsion cires-dans-eau, les cires pouvant être sous forme de particules de taille moyenne allant de 1 µm à 10 µm, et de préférence de 1 µm à 3,5 µm.
Dans un autre mode de réalisation de la composition selon l'invention, la cire peut être présente sous forme de microdispersion de cire, la cire étant sous forme de particules dont la taille moyenne est inférieure à 1 µm, et va notamment de 50 nm à 500 nm. Des microdispersions de cires sont décrites dans les documents EP-A-557196, EP-A-1048282.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1 % à 50 % en poids, par rapport au poids total de la composition, de préférence de 0,5 % à 40 % en poids, et mieux de 1 % à 30 % en poids.

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXINR" de Rhéox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0 à 60% (notamment 0,01 % à 60 %) en poids, par rapport au poids total de la composition, de préférence allant de 0,5 à 45 % en poids, et mieux allant de 2 % à 30 % en poids, dans la composition.

La composition selon l'invention peut également comprendre un milieu aqueux, constituant une phase aqueuse, qui peut être la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.
La phase aqueuse (eau et éventuellement le solvant organique miscible à l'eau) peut être présente, en une teneur allant de 1 % à 99 % en poids, par rapport au poids total de la composition, de préférence de 3 % à 90 % en poids, et mieux de 5 % à 80 % en poids.

La composition selon l'invention peut contenir des agents tensioactifs émulsionnants présents notamment en une proportion allant de 1 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les actifs cosmétiques ou dermatologiques comme par exemple des émollients, des hydratants, des vitamines, des filtres solaires, et leurs mélanges. Ces additifs peuvent être présents dans la composition en une teneur allant de 0 à 20% (notamment de 0,01 à 20 %) du poids total de la composition et mieux de 0,01 à 10% (si présents).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

L'invention est illustrée plus en détail dans les exemples suivants.

### Exemple 1 :

On a préparé un mascara ayant la composition suivante :
- Résine de polyamide avec groupes ester terminaux vendu sous la dénomination "UNICLEAR^{®} 100" par la société Arizona Chemical 0,5 g
- Cire de carnauba 2,9 g
- Cire d'abeille 3,6 g
- Cire de paraffine 11,4 g
- Amino-2 méthyl-2 propanediol-1,3 0,5 g
- Triéthanolamine 2,4 g
- Acide stéarique 5,8 g
- Polymères non-ioniques hydrosolubles 4,3 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Pigments 5,4 g
- Conservateurs qs
- Eau qsp 100 g

Ce mascara s'applique facilement, adhère bien sur les cils pendant et après l'application ; ces derniers sont maquillés rapidement. Il confère une charge instantanée des cils.

### Exemple 2 :

On a préparé un mascara ayant la composition suivante :
- Résine de polyamide avec groupes ester terminaux vendu sous la dénomination "UNICLEAR^{®} 100" par la société Arizona Chemical 0,5 g
- Cire de carnauba 4,7 g
- Cire d'abeille 4,9 g
- Cire de paraffine 2,3 g
- Hydroxyéthylcellulose réticulée par l'épichlorhydrine quaternisée par la triméthylamine (JR 400 de la société UNION CARBIDE) 0,1 g
- Polyméthacrylate de sodium (Darvan 7 de la société VANDERBILT) 0,25 g MA
- desoxyribonucléate de sodium 0,2 g
- eau 8,4 g
- alcool éthylique 2 g
- Bentonite 5,3 g
- carbonate de propylène 1,7 g
- Copolymère vinylpyrrolidone/1-eicosène 2 g
- Copolymère acétate de vinyle/stéarate d'allyle (65/35) (Mexomère PQ de CHIMEX) 2,2 g
- Polylaurate de vinyle (Mexomère PP de CHIMEX) 0,7 g
- amidon de riz 1,5 g
- Pigments 4,2 g
- Conservateurs qs
- isododécane qsp 100 g

Ce mascara waterproof adhère bien sur les cils pendant et après l'application. Il confère aux cils une charge instantanée et permet de les maquiller rapidement.

## Revendications

1. Composition comprenant, dans un milieu physiologiquement acceptable contenant une phase grasse, :
- (i) un premier polymère de masse moléculaire moyenne en poids inférieure à 100 000, choisi parmi les polymères de formule (I') suivante et leurs mélanges : 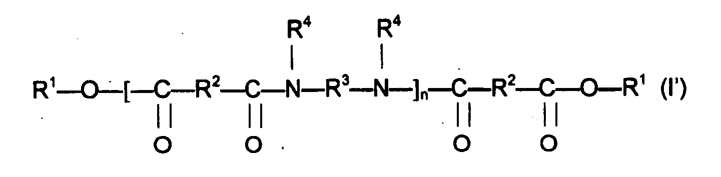 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que au moins 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène,
- (ii) un polymère filmogène anionique,
- (iii) un polymère filmogène cationique,
lesdits polymères filmogènes anionique et cationique étant différents dudit premier polymère.

2. Composition selon la revendication 1, **caractérisée par le fait que** la masse molaire moyenne du premier polymère est inférieure à 50 000.

3. Composition selon l'une quelconque des revendications précédentes, **caractérisée par le fait que** la masse moléculaire moyenne en poids du premier polymère va de 2 000 à 20 000 et mieux de 2 000 à 10 000.

4. Composition selon l'une des revendications 1 à 3, **caractérisée par le fait que** R¹ est un groupe alkyle en C₁₂ à C₂₂.

5. Composition selon l'une des revendications à 4, **caractérisée par le fait que** R² sont des groupes ayant de 30 à 42 atomes de carbone.

6. Composition selon l'une quelconque des revendications précédentes, **caractérisée par le fait que** le premier polymère est présent en une teneur allant de 0,01 % à 10 % en poids, par rapport au poids total de la composition, de préférence allant de 0,05 % à 5 % en poids, et mieux allant de 0,1 % à 3 % en poids.

7. Composition selon l'une quelconque des revendications précédentes, **caractérisée par le fait que** le polymère filmogène anionique est choisi parmi :
- les polymères comportant des motifs carboxyliques dérivant de monomères mono ou diacides carboxyliques insaturés de formule (I) : 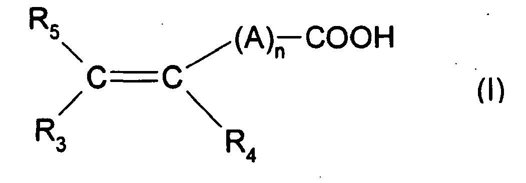 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle,
- les polymères comprenant des motifs dérivant d'acide sulfonique tels que des motifs vinylsulfonique, styrènesulfonique, acrylamido alkylsulfonique, et les polyesters sulfoniques,
- et leurs mélanges:

8. Composition selon l'une quelconque des revendications précédentes, **caractérisée en ce que** le polymère filmogène anionique est choisi parmi :
A) les homo- ou copolymères d'acide acrylique ou méthacrylique ou leurs sels, les sels de sodium des copolymères d'acide acrylique et d'acrylamide, les sels de sodium d'acides polyhydroxycarboxyliques ;
B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ et les terpolymères de vinylpyrrolidone, d'acide acrylique et de méthacrylate d'alkyle en C₁-C₂₀;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées ;
E) les polyacrylamides comportant des groupements carboxylates,
F) l'acide désoxyribonucléique;
G) les copolymères d'au moins un diacide carboxylique, d'au moins un diol et d'au moins un monomère aromatique bifonctionnel portant un groupement -SO₃M avec M représentant un atome d'hydrogène, un ion ammonium NH₄⁺ ou un ion métallique ;
- et leurs mélanges.

9. Composition selon l'une quelconque des revendications précédentes, **caractérisée en ce que** le polymère filmogène anionique est choisi parmi :
- les homopolymères d'acide acrylique ou méthacrylique ;
- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié.
- les copolymères d'acide méthacrylique et de méthacrylate de méthyle ;
- les copolymères d'acide méthacrylique et d'acrylate d'éthyle ;
- les terpolymères de vinylpyrrolidone/acide acrylique/méthacrylate de lauryle ;
- les copolymères acétate de vinyle/acide crotonique ;
- les terpolymères acétate de vinyle/acide crotonique/polyéthylèneglycol ;
- les sulfopolyesters obtenus par condensation de di-éthylèneglycol, de cyclohexane di-méthanol, d'acide isophtalique, d'acide sulfoisophtalique,
- et leurs mélanges.

10. Composition selon l'une quelconque des revendications précédentes, **caractérisée par le fait que** le polymère filmogène anionique est choisi parmi les polymères anioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale.

11. Composition selon la revendication 10, **caractérisée par le fait que** le polymère siliconé greffé est choisi parmi les polymères siliconés comportant dans leur structure le motif de formule (III) suivant : 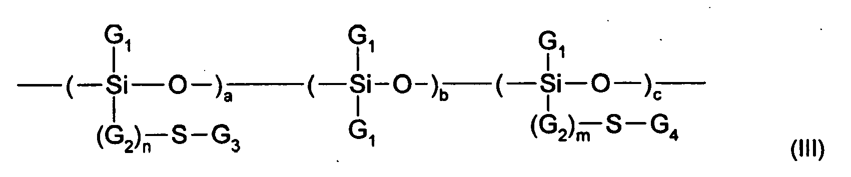 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

12. Composition selon la revendication 11, **caractérisée par le fait que** le motif de formule (III) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀ ;

13. Composition selon la revendication 11 ou 12, **caractérisée par le fait que** le motif de formule (III) présente simultanément les caractéristiques suivantes :
- les radicaux G₁ désignent un radical méthyle ;
- n est non nul, et les radicaux G₂ représentent un radical propylène;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins le (méth)acrylate d'isobutyle ou de méthyle.

14. Composition selon l'une quelconque des revendications précédentes, **caractérisée par le fait que** le polymère filmogène cationique est choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quaternisés ou non, les polymères quaternaires de vinylpyrrolidone et de vinylimidazole, les polyaminoamines et leurs mélanges.

15. Composition selon l'une quelconque des revendications précédentes, **caractérisée par le fait que** le polymère filmogène anionique est un polyméthacrylate de sodium.

16. Composition selon l'une quelconque des revendications précédentes, **caractérisée par le fait que** le polymère filmogène cationique est une hydroxyalkyl(C1-C4)cellulose comportant des groupements ammonium quaternaires.

17. Composition selon l'une quelconque des revendications précédentes, **caractérisée par le fait que** le polymère filmogène cationique est présent en une teneur allant de 0.01% à 20 % en poids; de préférence de 0,01 % à 15 % en poids, et encore plus préférentiellement de 0,05 % à 5 % en poids, du poids total de la composition.

18. Composition selon l'une quelconque des revendications précédentes, **caractérisée par le fait que** le polymère filmogène anionique est présent en une teneur allant de 0,01 à 20% en poids du poids total de la composition, de préférence de 0,05 à 15% en poids et encore plus préférentiellement 0,1 % à 7 % en poids.

19. Composition selon l'une quelconque des revendications précédentes, **caractérisée par le fait qu'**elle comprend, en outre, une cire.

20. Composition selon la revendication 19, **caractérisée par le fait que** la cire est choisie dans le groupe formé par la cire d'abeilles, la cire de lanoline, les cires d'insectes de Chine, la cire de riz, la cire de Carnauba, la cire de Candellila, la cire d'Ouricury, la cire de fibres de liège, la cire de canne à sucre, la cire du Japon et la cire de sumac, la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C, les cires obtenues par hydrogénation catalytique d'huiles animales ou végétales ayant des chaînes grasses, linéaires ou ramifiées, en C8-C32, les cires de silicone, les cires fluorées, et leurs mélanges.

21. Composition selon la revendications 19 ou 20, **caractérisée par le fait que** la cire est présente en une teneur allant de 0,1 % à 50 % en poids, par rapport au poids total de la composition, dé préférence de 0,5 % à 40 % en poids, et mieux de 1 % à 30 % en poids.

22. Composition selon l'une quelconque des revendications précédentes, **caractérisée par le fait que** la phase grasse comprend au moins une huile choisie dans le groupe formé par les huiles d'origine minérale, animale, végétale ou synthétique, hydrocarbonées, fluorées et/ou siliconées, seules ou en mélange.

23. Composition selon l'une quelconque des revendications précédentes, **caractérisée par le fait que** la phase grasse comprend au moins une huile volatile.

24. Composition selon l'une quelconque des revendications précédentes, **caractérisée par le fait que** la phase grasse comprend une huile volatile choisie parmi les huiles volatiles hydrocarbonées ayant de 8 à 16 atomes de carbone.

25. Composition selon l'une quelconque des revendications précédentes, **caractérisée par le fait que** la composition comprend une phase aqueuse contenant de l'eau ou un mélange d'eau et de solvant organique miscible à l'eau.

26. Composition selon l'une quelconque des revendications précédentes, **caractérisée par le fait que** la composition contient au moins une matière colorante.

27. Composition selon la revendication 26, **caractérisée par le fait que** la matière colorante est choisie parmi les pigments, les nacres, les colorants hydrosolubles, les colorants liposolubles, et leurs mélanges.

28. Composition selon l'une quelconque des revendications précédentes, **caractérisée par le fait que** la composition se présente sous forme de mascara, de produit pour les sourcils, de produit pour les cheveux.

29. Mascara comprenant une composition selon l'une quelconque des revendications 1 à 27.

30. Procédé de maquillage ou de soin non thérapeutique des matières kératiniques, notamment des fibres kératiniques, comprenant l'application sur les matières kératiniques d'une composition selon l'une quelconque des revendications précédentes.

31. Utilisation d'une composition selon l'une quelconque des revendications 1 à 28 pour l'obtention d'un dépôt adhérent sur les matières kératiniques et/ou pour obtenir un maquillage rapide des matières kératiniques.

32. Utilisation d'un mascara selon la revendication 29 pour épaissir les cils.

## Claims

1. Composition comprising, in a physiologically acceptable medium containing a fatty phase:
- (i) a first polymer with a weight-average molecular mass of less than 100 000, chosen from the polymers of formula (I') below, and mixtures thereof: 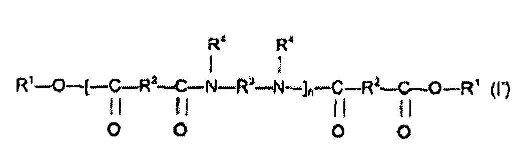 in which n denotes a number of amide units such that the number of ester groups represents from 10% to 50% of the total number of ester and amide groups; R¹ is, independently in each case, an alkyl or alkenyl group containing at least 4 carbon atoms; R² represents, independently in each case, a C₄ to C₄₂ hydrocarbon-based group, on condition that at least 50% of the groups R² represent a C₃₀ to C₄₂ hydrocarbon-based group; R³ represents, independently in each case, an organic group containing at least 2 carbon atoms, hydrogen atoms and optionally one or more oxygen or nitrogen atoms; and R⁴ represents, independently in each case, a hydrogen atom, a C₁ to C₁₀ alkyl group or a direct bond to R³ or to another R⁴, such that the nitrogen atom to which R³ and R⁴ are both attached forms part of a heterocyclic structure defined by R⁴-N-R³, with at least 50% of the groups R⁴ representing a hydrogen atom,
- (ii) an anionic film-forming polymer,
- (iii) a cationic film-forming polymer,
the said anionic and cationic film-forming polymers being different from the said first polymer.

2. Composition according to Claim 1, **characterized in that** the average molar mass of the first polymer is less than 50 000.

3. Composition according to either of the preceding claims, **characterized in that** the weight-average molecular mass of the first polymer ranges from 2000 to 20 000 and better still from 2000 to 10 000.

4. Composition according to one of Claims 1 to 3, **characterized in that** R¹ is a C₁₂ to C₂₂ alkyl group.

5. Composition according to one of Claims 1 to 4, **characterized in that** the radicals R² are groups containing from 30 to 42 carbon atoms.

6. Composition according to any one of the preceding claims, **characterized in that** the first polymer is present in a content ranging from 0.01% to 10% by weight, preferably ranging from 0.05% to 5% by weight and better still ranging from 0.1% to 3% by weight, relative to the total weight of the composition.

7. Composition according to any one of the preceding claims, **characterized in that** the anionic film-forming polymer is chosen from:
- polymers comprising carboxylic units derived from unsaturated monocarboxylic or dicarboxylic acid monomers of formula (I): 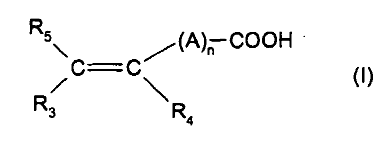 in which n is an integer from 0 to 10, A denotes a methylene group, optionally connected to the carbon atom of the unsaturated group or to the neighbouring methylene group when n is greater than 1 via a heteroatom such as oxygen or sulfur, R₅ denotes a hydrogen atom or a phenyl or benzyl group, R₃ denotes a hydrogen atom or a lower alkyl or carboxyl group, and R₄ denotes a hydrogen atom, a lower alkyl group or a
- CH₂-COOH, phenyl or benzyl group,
- polymers comprising units derived from sulfonic acid, such as vinylsulfonic, styrenesulfonic and acrylamidoalkylsulfonic units, and sulfonic polyesters, and
- mixtures thereof.

8. Composition according to any one of the preceding claims, **characterized in that** the anionic film-forming polymer is chosen from:
A) homo- or copolymers of acrylic or methacrylic acid or salts thereof, the sodium salts of copolymers of acrylic acid and of acrylamide, and the sodium salts of polyhydroxycarboxylic acids;
B) copolymers of acrylic or methacrylic acids with a monoethylenic monomer such as ethylene, styrene, vinyl esters and acrylic or methacrylic acid esters, optionally grafted onto a polyalkylene glycol such as polyethylene glycol; copolymers of this type comprising in their chain an optionally N-alkylated and/or hydroxyalkylated acrylamide unit, copolymers of acrylic acid and of C₁-C₄ alkyl methacrylate and terpolymers of vinylpyrrolidone, of acrylic acid and of C₁-C₂₀ alkyl methacrylate;
C) copolymers derived from crotonic acid, such as those whose chain comprises vinyl acetate or propionate units and optionally other monomers such as allylic or methallylic esters, vinyl ether or vinyl ester of a saturated, linear or branched carboxylic acid containing a long hydrocarbon-based chain such as those comprising at least 5 carbon atoms, it being possible for these polymers to be optionally grafted;
D) polymers derived from maleic, fumaric or itaconic acids or anhydrides with vinyl esters, vinyl ethers, vinyl halides, phenylvinyl derivatives, acrylic acid and esters thereof; copolymers of maleic, citraconic or itaconic anhydrides and of an allylic or methallylic ester optionally comprising an acrylamide, methacrylamide, α-olefin, acrylic or methacrylic ester, acrylic or methacrylic acid or vinylpyrrolidone group in their chain, the anhydride functions are monoesterified or monoamidated;
E) polyacrylamides comprising carboxylate groups,
F) deoxyribonucleic acid;
G) copolymers of at least one dicarboxylic acid, of at least one diol and of at least one difunctional aromatic monomer bearing a group -SO₃M with M representing a hydrogen atom, an ammonium ion NH₄⁺ or a metal ion;
- and mixtures thereof.

9. Composition according to any one of the preceding claims, **characterized in that** the anionic film-forming polymer is chosen from:
- acrylic or methacrylic acid homopolymers;
- acrylic acid copolymers such as the acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives or acrylic acid and esters thereof, such as methyl vinyl ether/monoesterified maleic anhydride copolymers;
- copolymers of methacrylic acid and of methyl methacrylate;
- copolymers of methacrylic acid and of ethyl acrylate;
- terpolymers of vinylpyrrolidone/acrylic acid/lauryl methacrylate;
- vinyl acetate/crotonic acid copolymers;
- vinyl acetate/crotonic acid/polyethylene glycol terpolymers;
- sulfopolyesters obtained by condensation of diethylene glycol, cyclohexanedimethanol, isophthalic acid and sulfoisophthalic acid,
- and mixtures thereof.

10. Composition according to any one of the preceding claims, **characterized in that** the anionic film-forming polymer is chosen from anionic polymers of grafted silicone type comprising a polysiloxane portion and a portion consisting of a non-silicone organic chain, one of the two portions constituting the main chain of the polymer, the other being grafted onto the said main chain.

11. Composition according to Claim 10, **characterized in that** the grafted silicone polymer is chosen from silicone polymers whose structure comprises the unit of formula (III) below: 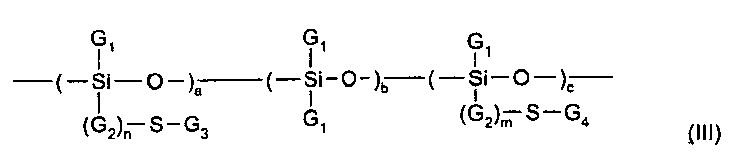 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one ethylenically unsaturated anionic monomer; G₄ represents a polymer residue resulting from the (homo)polymerization of at least one ethylenically unsaturated hydrophobic monomer; m and n are equal to 0 or 1; a is an integer ranging from 0 to 50; b is an integer which can be between 10 and 350, c is an integer ranging from 0 to 50; with the proviso that one of the parameters a and c is other than 0.

12. Composition according to Claim 11, **characterized in that** the unit of formula (III) has at least one of the following characteristics:
- the radicals G₁ denote a C₁-C₁₀ alkyl radical;
- n is non-zero and the radicals G₂ represent a divalent C₁-C₃ radical;
- G₃ represents a polymer radical resulting from the (homo)polymerization of at least one monomer such as an ethylenically unsaturated carboxylic acid;
- G₄ represents a polymer radical resulting from the (homo)polymerization of at least one monomer such as a C₁-C₁₀ alkyl (meth)acrylate.

13. Composition according to Claim 11 or 12,
**characterized in that** the unit of formula (III) simultaneously has the following characteristics:
- the radicals G₁ denote a methyl radical;
- n is non-zero and the radicals G₂ represent a propylene radical;
- G₃ represents a polymer radical resulting from the (homo)polymerization of at least acrylic acid and/or methacrylic acid;
- G₄ represents a polymer radical resulting from the (homo)polymerization of at least isobutyl or methyl (meth)acrylate.

14. Composition according to any one of the preceding claims, **characterized in that** the cationic film-forming polymer is chosen from quaternary cellulose ether derivatives, copolymers of cellulose with a water-soluble quaternary ammonium monomer, cyclopolymers, cationic polysaccharides, cationic silicone polymers, quaternized or non-quaternized vinylpyrrolidone-dialkylaminoalkyl acrylate or methacrylate copolymers, quaternary polymers of vinylpyrrolidone and of vinylimidazole, and polyaminoamides, and mixtures thereof.

15. Composition according to any one of the preceding claims, **characterized in that** the anionic film-forming polymer is a poly(sodium methacrylate).

16. Composition according to any one of the preceding claims, **characterized in that** the cationic film-forming polymer is a hydroxy(C₁-C₄)alkylcellulose comprising quaternary ammonium groups.

17. Composition according to any one of the preceding claims, **characterized in that** the cationic film-forming polymer is present in a content ranging from 0.01% to 20% by weight, preferably from 0.01% to 15% by weight and even more preferentially from 0.05% to 5% by weight, relative to the total weight of the composition.

18. Composition according to any one of the preceding claims, **characterized in that** the anionic film-forming polymer is present in a content ranging from 0.01% to 20% by weight, preferably from 0.05% to 15% by weight and even more preferentially from 0.1% to 7% by weight, relative to the total weight of the composition.

19. Composition according to any one of the preceding claims, **characterized in that** it also comprises a wax.

20. Composition according to Claim 19, **characterized in that** the wax is chosen from the group formed by beeswax, lanolin wax, Chinese insect waxes, rice wax, carnauba wax, candelilla wax, ouricury wax, cork fibre wax, sugar cane wax, Japan wax, sumach wax, montan wax, microcrystalline waxes, paraffin waxes, ozokerites, ceresin wax, lignite wax, polyethylene waxes and the waxes obtained by Fisher-Tropsch synthesis, fatty acid esters and glycerides that are solid at 40°C, the waxes obtained by catalytic hydrogenation of animal or plant oils containing linear or branched C₈-C₃₂ fatty chains, silicone waxes and fluoro waxes, and mixtures thereof.

21. Composition according to Claim 19 or 20, **characterized in that** the wax is present in a content ranging from 0.1% to 50% by weight, preferably from 0.5% to 40% by weight and better still from 1% to 30% by weight, relative to the total weight of the composition.

22. Composition according to any one of the preceding claims, **characterized in that** the fatty phase comprises at least one oil chosen from the group formed by hydrocarbon-based oils, fluoro oils and/or silicone oils of mineral, animal, plant or synthetic origin, alone or as a mixture.

23. Composition according to any one of the preceding claims, **characterized in that** the fatty phase comprises at least one volatile oil.

24. Composition according to any one of the preceding claims, **characterized in that** the fatty phase comprises a volatile oil chosen from hydrocarbon-based volatile oils containing from 8 to 16 carbon atoms.

25. Composition according to any one of the preceding claims, **characterized in that** the composition comprises an aqueous phase containing water or a mixture of water and of water-miscible organic solvent.

26. Composition according to any one of the preceding claims, **characterized in that** the composition contains at least one dyestuff.

27. Composition according to Claim 26, **characterized in that** the dyestuff is chosen from pigments, nacres, water-soluble dyes and liposoluble dyes, and mixtures thereof.

28. Composition according to any one of the preceding claims, **characterized in that** the composition is in the form of a mascara, a product for the eyebrows or a product for the hair.

29. Mascara comprising a composition according to any one of Claims 1 to 27.

30. Non-therapeutic makeup or care process for keratin materials, especially keratin fibres, comprising the application to the keratin materials of a composition according to any one of the preceding claims.

31. Use of a composition according to any one of Claims 1 to 28, to obtain a deposit that adheres to keratin materials and/or to obtain a fast makeup result on keratin materials.

32. Use of a mascara according to Claim 29, to thicken the eyelashes.

## Patentansprüche

1. Zusammensetzung, die in einem eine Fettphase enthaltenden, physiologisch akzeptablen Medium enthält:
(i) ein erstes Polymer mit einer gewichtsmittleren Molmasse unter 100 000, das unter den Polymeren der folgenden Formel (I') und deren Gemischen ausgewählt ist: 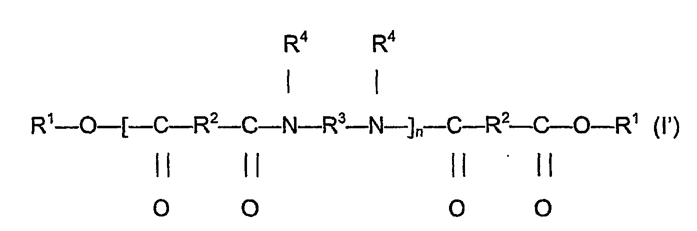 wobei n die Anzahl der Amideinheiten bezeichnet und so ausgewählt ist, dass die Anzahl der Estergruppen 10 bis 50 % der Gesamtzahl der Ester- und Amidgruppen ausmacht; die Gruppen R¹ jeweils unabhängig voneinander Alkyl- oder Alkenylgruppen mit mindestens 4 Kohlenstoffatomen bedeuten; die Gruppen R² jeweils unabhängig voneinander C₄-₄₂-Kohlenwasserstoffgruppen bedeuten, mit der Maßgabe, dass mindestens 50 % der Gruppen R² C₃₀₋₄₂-Kohlenwasserstoffgruppen sind; die Gruppen R³ jeweils unabhängig voneinander organische Gruppen bedeuten, die mindestens zwei Kohlenstoffatome, Wasserstoffatome und gegebenenfalls ein oder mehrere Sauerstoffatome oder Stickstoffatome enthalten; und die Gruppen R⁴ jeweils unabhängig voneinander Wasserstoff, C₁₋₁₀-Alkyl oder eine direkte Bindung zu R³ oder einer weiteren Gruppe R⁴ bedeuten, die so gebildet wird, dass das Stickstoffatom, an das die Gruppen R³ und R⁴ gleichzeitig gebunden sind, Teil einer heterocyclischen Struktur ist, die durch R⁴-N-R³ definiert ist, wobei mindestens 50 % der Gruppen R⁴ Wasserstoff bedeuten,
(ii) ein anionisches filmbildendes Polymer,
(iii) ein kationisches filmbildendes Polymer,
wobei das anionische und das kationische filmbildende Polymer von dem ersten Polymer verschieden sind.

2. Zusammensetzung nach Anspruch 1, **dadurch gekennzeichnet, dass** die mittlere Molmasse des ersten Polymers unter 50 000 liegt.

3. Zusammensetzung nach einem der vorhergehenden Ansprüche, **dadurch gekennzeichnet, dass** die gewichtsmittlere Molmasse des ersten Polymers im Bereich von 2 000 bis 20 000 und besser 2 000 bis 10 000 liegt.

4. Zusammensetzung nach einem der Ansprüche 1 bis 3, **dadurch gekennzeichnet, dass** R¹ eine C₁₂₋₂₂-Alkylgruppe ist.

5. Zusammensetzung nach einem der Ansprüche 1 bis 4, **dadurch gekennzeichnet, dass** die Gruppen R² Gruppen mit 30 bis 42 Kohlenstoffatomen bedeuten.

6. Zusammensetzung nach einem der vorhergehenden Ansprüche, **dadurch gekennzeichnet, dass** das erste Polymer in einem Mengenanteil von 0,01 bis 10 Gew.-%, bezogen auf das Gesamtgewicht der Zusammensetzung, vorzugsweise von 0,05 bis 5 Gew.-% und besser 0,1 bis 3 Gew.-% enthalten ist.

7. Zusammensetzung nach einem der vorhergehenden Ansprüche, **dadurch gekennzeichnet, dass** das anionische filmbildende Polymer ausgewählt ist unter:
- Polymeren mit Carboxygruppen, die von ungesättigten Mono- oder Dicarbonsäuremonomeren der Formel (I) abgeleitet sind: 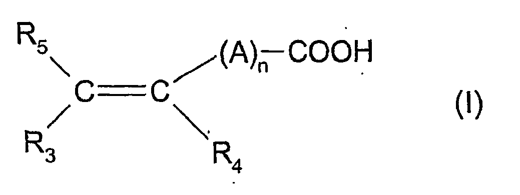 wobei in der Formel bedeuten:
n Null oder eine ganze Zahl von 1 bis 10, A eine Methylengruppe, die gegebenenfalls an das Kohlenstoffatom der ungesättigten Gruppe oder, falls n größer 1 ist, an die benachbarte Methylengruppe über ein Heteroatom, wie Sauerstoff oder Schwefel, gebunden ist, R₅ ein Wasserstoffatom, eine Phenylgruppe oder eine Benzylgruppe, R₃ ein Wasserstoffatom, eine niedere Alkylgruppe oder eine Carboxygruppe und R₄ ein Wasserstoffatom, eine niedere Alkylgruppe, eine Gruppe -CH₂-COOH, eine Phenylgruppe oder eine Benzylgruppe;
- Polymeren, die von einer Sulfonsäure abgeleitete Einheiten enthalten, wie beispielsweise Vinylsulfonsäureeinheiten, Styrolsulfonsäureeinheiten oder Acrylamidoalkylsulfonsäureeinheiten, oder Sulfopolyestern;
- und deren Gemischen.

8. Zusammensetzung nach einem der vorhergehenden Ansprüche, **dadurch gekennzeichnet, dass** das anionische filmbildende Polymer ausgewählt ist unter:
A) Homo- oder Copolymeren von Acrylsäure oder Methacrylsäure oder deren Salzen, den Natriumsalzen von Copolymeren von Acrylsäure und Acrylamid, den Natriumsalzen von Polyhydroxycarbonsäuren;
B) Copolymeren von Acrylsäure oder Methacrylsäure mit einem monoethylenisch ungesättigten Monomer, wie Ethylen, Styrol, Vinylestern, Acrylsäure- oder Methacrylsäureestern, die gegebenenfalls auf ein Polyalkylenglycol, wie Polyethylenglycol, gepfropft sind; Copolymeren dieses Typs, die in ihrer Kette eine Acrylamideinheit aufweisen, die gegebenenfalls N-alkyliert und/oder hydroxyalkyliert ist; Copolymeren von Acrylsäure und C₁₋₄-Alkylmethacrylat und Terpolymeren von Vinylpyrrolidon, Acrylsäure und C₁₋₂₀-Alkylmethacrylat;
C) Copolymeren, die von Crotonsäure abgeleitet sind, wie Copolymeren, die in ihrer Kette Vinylacetat- oder Vinylpropionat-Einheiten und gegebenenfalls weitere Monomere aufweisen, wie Allylester oder Methallylester, Vinylether oder Vinylester einer gesättigten, geradkettigen oder verzweigten Carbonsäure mit langer Kohlenwasserstoffkette, wie Carbonsäuren mit mindestens 5 Kohlenstoffatomen, wobei diese Polymere gegebenenfalls gepfropft sein können;
D) Polymeren, die von Maleinsäure, Fumarsäure, Itaconsäure oder deren Anhydriden mit Vinylestern, Vinylethern, Vinylhalogeniden, Phenylvinylderivaten, Acrylsäure und ihren Estern abgeleitet sind; Copolymeren von Maleinsäureanhydrid, Citraconsäureanhydrid oder Itaconsäureanhydrid mit einem Allylester oder einem Methallylester, die in ihrer Kette gegebenenfalls eine der folgenden Gruppen enthalten: Acrylamid, Methacrylamid, α-Olefin, Acrylester, Methacrylester, Acrylsäure, Methacrylsäure oder Vinylpyrrolidon, wobei die Anhydridgruppen einfach verestert sind oder als einfaches Amid vorliegen;
E) Polyacrylamiden mit Carboxylatgruppen;
F) Desoxyribonucleinsäure;
G) Copolymeren mindestens einer Dicarbonsäure, mindestens eines Diols und mindestens eines bifunktionellen aromatischen Monomers mit einer Gruppe -SO₃M, worin M ein Wasserstoffatom, ein Ammoniumion NH₄⁺ oder ein Metallion bedeutet, und den Gemischen dieser Verbindungen.

9. Zusammensetzung nach einem der vorhergehenden Ansprüche, **dadurch gekennzeichnet, dass** das anionische filmbildende Polymer ausgewählt ist unter:
- Homopolymeren von Acrylsäure oder Methacrylsäure,
- Copolymeren von Acrylsäure, wie dem Acrylsäure/Ethylacrylat/N-*tert*-Butylacrylamid-Terpolymer,
- Copolymeren, die von Crotonsäure abgeleitet sind, wie Vinylacetat/Vinyl-*tert*-butylbenzoat/Crotonsäure-Terpolymeren und Crotonsäure/Vinylacetat/Vinylneododecanoat-Terpolymeren,
- Polymeren, die von Maleinsäure, Fumarsäure, Itaconsäure oder deren Anhydriden mit Vinylestern, Vinylethern, Vinylhalogeniden, Phenylvinylderivaten, Acrylsäure und ihren Estern abgeleitet sind, wie den Copolymeren von Methylvinylether und einfach verestertem Maleinsäureanhydrid,
- Copolymeren von Methacrylsäure und Methylmethacrylat,
- Copolymeren von Methacrylsäure und Ethylacrylat,
- Vinylpyrrolidon/Acrylsäure/Laurylmethacrylat-Terpolymeren,
- Vinylacetat/Crotonsäure-Copolymeren,
- Vinylacetat/Crotonsäure/Polyethylenglycol-Terpolymeren,
- Sulfopolyestern, die durch Kondensation von Diethylenglycol, Cyclohexandimethanol, Isophthalsäure und Sulfoisophthalsäure erhalten werden, und
- den Gemischen dieser Verbindungen.

10. Zusammensetzung nach einem der vorhergehenden Ansprüche, **dadurch gekennzeichnet, dass** das anionische filmbildende Polymer unter den anionischen Polymeren vom Typ der gepfropften Siliconpolymere ausgewählt ist, die einen Polysiloxanteil und einen Teil aufweisen, der aus einer nicht siliconhaltigen organischen Kette besteht, wobei ein Teil die Hauptkette des Polymers bildet und der andere Teil auf die Hauptkette gepfropft ist.

11. Zusammensetzung nach Anspruch 10, **dadurch gekennzeichnet, dass** das gepfropfte Siliconpolymer unter den Siliconpolymeren ausgewählt ist, die in ihrer Struktur eine Einheit der folgenden Formel (III) aufweisen: 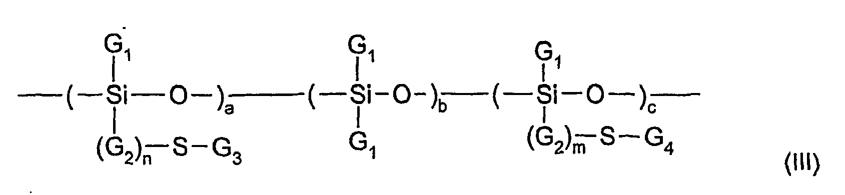 worin bedeuten:
die Gruppen G₁, die identisch oder voneinander verschieden sind, Wasserstoff oder eine Alkylgruppe mit 1 bis 10 Kohlenstoffatomen oder auch eine Phenylgruppe; die Gruppen G₂, die identisch oder voneinander verschieden sind, eine Alkylengruppe mit 1 bis 10 Kohlenstoffatomen; G₃ eine Polymergruppe, die das Ergebnis der (Homo)polymerisation mindestens eines anionischen Monomers mit ethylenischer Doppelbindung ist; G₄ eine Polymergruppe, die bei der (Homo)polymerisation mindestens eines hydrophoben Monomers mit ethylenischer Doppelbindung entsteht; m und n Null oder 1; a Null oder eine ganze Zahl von 1 bis 50; b eine ganze Zahl, die im Bereich von 10 bis 350 liegen kann; und c Null oder eine ganze Zahl von 1 bis 50; mit der Maßgabe, dass einer der Parameter a und c von Null verschieden ist.

12. Zusammensetzung nach Anspruch 11, **dadurch gekennzeichnet, dass** die Einheit der Formel (III) mindestens eine der folgenden Eigenschaften aufweist:
- die Gruppen G₁ bedeuten eine Alkylgruppe mit 1 bis 10 Kohlenstoffatomen,
- n ist nicht Null und die Gruppen G₂ bedeuten eine zweiwertige Gruppe mit 1 bis 3 Kohlenstoffatomen,
- G₃ bedeutet eine Polymergruppe, die das Ergebnis der (Homo)polymerisation mindestens eines Monomers vom Typ einer Carbonsäure mit ethylenischer Doppelbindung ist, und
- G₄ bedeutet eine Polymergruppe, die bei der (Homo)polymerisation mindestens eines Monomers vom Typ C₁₋₁₀-Alkyl(meth)acrylat gebildet wird.

13. Zusammensetzung nach Anspruch 11 oder 12, **dadurch gekennzeichnet, dass** die Einheit der Formel (III) gleichzeitig die folgenden Eigenschaften aufweist:
- die Gruppen G₁ bedeuten eine Methylgruppe,
- n ist nicht Null und die Gruppen G₂ bedeuten eine Propylengruppe,
- G₃ bedeutet eine Polymergruppe, die durch (Homo)polymerisation von zumindest Acrylsäure und/oder Methacrylsäure entsteht, und
- G₄ bedeutet eine Polymergruppe, die das Ergebnis der (Homo)polymerisation von zumindest Isobutyl(meth)acrylat oder Methyl-(meth)acrylat ist.

14. Zusammensetzung nach einem der vorhergehenden Ansprüche, **dadurch gekennzeichnet, dass** das kationische filmbildende Polymer ausgewählt ist unter den quartären Celluloseetherderivaten, Cellulosecopolymeren mit einem wasserlöslichen quartären Ammoniummonomer, Cyclopolymeren, kationischen Polysacchariden, kationischen Siliconpolymeren, quaternisierten oder nicht quaternisierten Copolymeren von Vinylpyrrolidon und Dialkylaminoalkylacrylat oder Dialkylaminoalkylmethacrylat, quartären Polymeren von Vinylpyrrolidon und Vinylimidazol, Polyaminoaminen und den Gemischen dieser Verbindungen.

15. Zusammensetzung nach einem der vorhergehenden Ansprüche, **dadurch gekennzeichnet, dass** das anionische filmbildende Polymer ein Natriumpolymethacrylat ist.

16. Zusammensetzung nach einem der vorhergehenden Ansprüche, **dadurch gekennzeichnet, dass** das kationische filmbildende Polymer eine Hydroxyalkyl(C₁₋₄)cellulose mit quartären Ammoniumgruppen ist.

17. Zusammensetzung nach einem der vorhergehenden Ansprüche, **dadurch gekennzeichnet, dass** das kationische filmbildende Polymer in einem Mengenanteil von 0,01 bis 20 Gew.-%, vorzugsweise von 0,01 bis 15 Gew.-% und noch bevorzugter von 0,05 bis 5 Gew.-% des Gesamtgewichts der Zusammensetzung vorliegt.

18. Zusammensetzung nach einem der vorhergehenden Ansprüche, **dadurch gekennzeichnet, dass** das anionische filmbildende Polymer in einer Menge von 0,01 bis 20 Gew.-% des Gesamtgewichts der Zusammensetzung, vorzugsweise 0,05 bis 15 Gew.-% und noch bevorzugter 0,1 bis 7 Gew.-% enthalten ist.

19. Zusammensetzung nach einem der vorhergehenden Ansprüche, **dadurch gekennzeichnet, dass** sie ferner ein Wachs enthält.

20. Zusammensetzung nach Anspruch 19, **dadurch gekennzeichnet, dass** das Wachs unter Bienenwachs, Lanolinwachs, Chinawachs, Reiswachs, Carnaubawachs, Candelillawachs, Ouricurywachs, Korkfaserwachs, Zuckerrohrwachs, Japanwachs und Sumachwachs, Montanwachs, mikrokristallinen Wachsen, Paraffinen, Ozokeriten, Ceresinwachs, Lignitwachs, Polyethylenwachsen, durch Fischer-Tropsch-Synthese hergestellten Wachsen, bei 40°C festen Estern von Fettsäuren und Glyceriden, durch katalytische Hydrierung tierischer oder pflanzlicher Öle hergestellten Wachsen mit linearen oder verzweigten C₈₋₃₂-Fettketten, Siliconwachsen, fluorierten Wachsen und deren Gemischen ausgewählt ist.

21. Zusammensetzung nach einem der Ansprüche 19 oder 20, **dadurch gekennzeichnet, dass** das Wachs in einem Mengenanteil von 0,1 bis 50 Gew.-%, bezogen auf das Gesamtgewicht der Zusammensetzung, vorzugsweise 0,5 bis 40 Gew.-% und besser 1 bis 30 Gew.-% vorliegt.

22. Zusammensetzung nach einem der vorhergehenden Ansprüche, **dadurch gekennzeichnet, dass** die Fettphase mindestens ein Öl enthält, das unter den Ölen auf Kohlenwasserstoffbasis, fluorierten Ölen und/oder Siliconölen mineralischer, tierischer, pflanzlicher oder synthetischer Herkunft oder deren Gemischen ausgewählt ist.

23. Zusammensetzung nach einem der vorhergehenden Ansprüche, **dadurch gekennzeichnet, dass** die Fettphase ferner mindestens ein flüchtiges Öl enthält.

24. Zusammensetzung nach einem der vorhergehenden Ansprüche, **dadurch gekennzeichnet, dass** die Fettphase ein flüchtiges Öl enthält, das unter den flüchtigen Kohlenwasserstoffölen mit 8 bis 16 Kohlenstoffatomen ausgewählt ist.

25. Zusammensetzung nach einem der vorhergehenden Ansprüche, **dadurch gekennzeichnet, dass** die Zusammensetzung eine wässrige Phase enthält, die Wasser oder ein Gemisch von Wasser und einem mit Wasser mischbaren Lösemittel enthält.

26. Zusammensetzung nach einem der vorhergehenden Ansprüche, **dadurch gekennzeichnet, dass** die Zusammensetzung mindestens ein Farbmittel enthält.

27. Zusammensetzung nach Anspruch 26, **dadurch gekennzeichnet, dass** das Farbmittel unter den Pigmenten, Perlglanzpigmenten, wasserlöslichen Farbstoffen, fettlöslichen Farbstoffen und deren Gemischen ausgewählt ist.

28. Zusammensetzung nach einem der vorhergehenden Ansprüche, **dadurch gekennzeichnet, dass** die Zusammensetzung als Mascara, Produkt für die Augenbrauen oder Produkt für die Haare vorliegt.

29. Mascara, die eine Zusammensetzung nach einem der Ansprüche 1 bis 27 enthält.

30. Verfahren zum Schminken oder für die nichttherapeutische Pflege von Keratinsubstanzen und insbesondere Keratinfasern, das umfasst, eine Zusammensetzung nach einem der vorhergehenden Ansprüche auf die Keratinsubstanzen aufzubringen.

31. Verwendung einer Zusammensetzung nach einem der Ansprüche 1 bis 28, um auf den Keratinsubstanzen ein haftendes Depot zu bilden und/oder die Keratinsubstanzen schnell zu schminken.

32. Verwendung einer Mascara nach Anspruch 29. um die Wimpern dicker werden zu lassen.
